# EUROPÄISCHE PATENTSCHRIFT

(11) **EP 0 726 464 B1**
(45) Veröffentlichungstag und Bekanntmachung des Hinweises auf die Patenterteilung: **04.04.2001**
(21) Anmeldenummer: 96100267.2
(22) Anmeldetag: 10.01.1996
(51) Int. Cl.: G01N 33/58, G01N 33/543, G01N 33/78, G01N 33/74

(54) **Kompetitiver Immuntest unter Verwendung komplexierter Analytderivate**
Competitive immune test using analyte-derivate complexes
Essai immunologique compétitif utilisant des complexes d'analytes-dérivés

(30) Priorität: 09.02.1995 DE 19504198
(43) Veröffentlichungstag der Anmeldung: 14.08.1996
(73) Patentinhaber: Dade Behring Marburg GmbH, 35001 Marburg (DE)
(72) Erfinder: Neuenhofer, Stephan, Dr., D-35037 Marburg (DE); Skrzipczyk, Heinz-Jürgen, Dr., D-63263 Zeppelinheim (DE); Molz, Peter, Dr., D-55118 Mainz (DE); Käsmarker, Reinhard, D-35041 Marburg (DE)

(56) Entgegenhaltungen:
- EP-A- 0 303 284
- WO-A-89/06363

## Beschreibung

Die Erfindung betrifft ein Verfahren zum Nachweis eines Analyten in einem kompetitiven Immuntest in Gegenwart eines Analytderivaten, sowie einen Kit zur Durchführung dieses Nachweisverfahrens.

Immunologische Nachweisverfahren haben in der in vitro Diagnostik eine hohe Bedeutung erlangt. Ursache hierfür ist, daß sie hochspezifisch und äußerst empfindlich sind. Zudem zeichnen sich diese Assays durch eine einfache Handhabung aus. Die Nachweisverfahren beruhen auf der immunologischen Wechselwirkung zwischen dem nachzuweisenden Analyten und seinem Bindungspartner bzw. -partnern.

Im Falle der Sandwich-Assays wird der Analyt von zwei verschiedenen Antikörpern sandwichartig gebunden. Einer der beiden Antikörper trägt eine Markierung (Label, Marker), wodurch seine Konzentration bestimmt werden kann. Im Falle kleiner Analyte scheidet das Sandwich-Verfahren aus, da aus sterischen Gründen nicht gleichzeitig zwei verschiedene Antikörper am Analyten binden können. Hier finden in der Regel die kompetitiven Assays Anwendung. Dabei konkurrieren der Analyt und ein synthetisches Derivat des Analyten um die Bindungsstellen des Antikörpers. Markiert wird in der Regel entweder das Analytderivat (klassisch kompetitives Verfahren) oder der Antikörper.

Häufig wird beim klassisch kompetitiven Verfahren der Antikörper an eine Festphase gebunden, während bei der Methode mit markiertem Antikörper das Analytderivat immobilisiert wird (solid phase antigen techniques, z.B. SPALT = solid phase antigen luminescencetechnique).

Unter den kompetitiven Testverfahren haben die Verfahren mit markierten Antikörpern besondere Bedeutung bei der Bestimmung des frei vorliegenden Anteils eines Analyten. erlangt. Dabei bedeutet "frei vorliegend", daß diese Analyte als Liganden nicht von ihren natürlichen Rezeptoren, die beispielsweise im Serum vorkommen können, gebunden sind. Derartige Verfahren haben beispielsweise bei der Bestimmung von freiem Triiodthyronin (FT3) und freiem Thyroxin (FT4) Bedeutung erlangt. Um den Anteil an Analyt unabhängig von der Konzentration in der Probe vorkommender Bindungsproteine (beispielsweise Serumproteine) bestimmen zu können, muß das Analytderivat bei der Bindung an Festphasen seine Eigenschaften derart verändern, daß es zwar noch mit dem Antikörper, nicht aber - oder nur unwesentlich - mit dem Bindungsprotein interagieren kann. Ein derartiges Verfahren wurde als ein 1-Schritt-Test beispielsweise in EP-A-0 103 605 beschrieben.

Dieses Verfahren unter Verwendung markierter Antikörper hat insbesondere den Vorteil der leichten Tracerherstellung, da die Markierung eines Antikörpers in den meisten Fällen unproblematisch ist. Sie ist daher gegenüber dem klassisch kompetitiven Verfahren auch dann noch vorzuziehen, wenn ein Analyttracer zur Verfügung steht, der das obengenannte, gewünschte Bindungsverhalten zeigt (EP-A-0 026 103).

Bei der Durchführung des vorstehend genannten Verfahrens bereitet die Wahl eines Analytderivaten mit geeigneter Affinität zum Antikörper häufig Probleme. Diese ergeben sich daraus, daß die Affinität des Analytderivaten zum Antikörper für ein aussagekräftiges Testergebnis in einem bestimmten Verhältnis zur Affinität des Analyten zum Antikörper stehen muß (vgl. EP-A-0 254 929, EP-A-0 324 540, EP-A-0 303 284). Ist beispielsweise die Affinität des Analytderivaten zum Antikörper zu hoch, so wird das Reaktionsgleichgewicht im Testansatz zu sehr in Richtung des Antikörper-Analyt-Derivat-Komplexes verschoben, was zu einer Beeinträchtigung der Aussagekraft des Testes führt. Um diesem Nachteil abzuhelfen, kann eine Vorinkubation des Analyten mit dem Antikörper durchgeführt werden (vgl. EP-A- 0 182 385). Ist dagegen die Affinität des Analytderivates zu niedrig, kann zunächst eine Vorinkubation von Analytderivat und Antikörper von Nutzen sein. Nachteilig ist in beiden Fällen jedoch die Notwendigkeit, daß der Test in zwei Schritten durchgeführt werden muß. Eine Möglichkeit, diesen Nachteil zu umgehen, besteht darin, den Analytderivaten chemisch zu modifizieren, um eine geeignete Affinität zum Antikörper herzustellen. Jedoch ist auch ein derartiges Verfahren aufwendig und bei kleinen Analytderivaten oft mit Schwierigkeiten verbunden oder gar nicht durchführbar.

Der vorliegenden Erfindung lag somit die Aufgabe zugrunde, ein Verfahren zum Nachweis eines Analyten in kompetitiven Immuntests bereitzustellen, das die vorstehend beschriebenen Nachteile überwindet. Diese Aufgabe wird durch die in den Ansprüchen bereitgestellten Ausführungsformen gelöst.

Somit betrifft die Erfindung ein Verfahren zum Nachweis eines Analyten in einem kompetitiven Immuntest, das dadurch gekennzeichnet ist, daß man folgende Schritte durchführt:
(a) Inkubation von einem Analytderivat mit einer den Analyten enthaltenden Probe sowie einem für Analyt und Analytderivat spezifischen ersten Rezeptormolekül, wobei der Inkubationsansatz ferner ein zweites Rezeptormolekül enthält, das spezifisch das Analytderivat oder das Analytderivat und den Analyten bindet;
(b) Abtrennung des nicht an das Analytderivat gebundenen ersten Rezeptormoleküls; oder
(b') Abtrennung des nicht an das erste Rezeptormolekül gebundenen Analytderivats; und
(c) Nachweis des an das Analytderivat gebundenen ersten Rezeptormoleküls oder des an das erste Rezeptormolekül gebundenen Analytderivats.

Der vorgenannte Schritt (b) wird durchgeführt, wenn das Analytderivat an eine Festphase gebunden ist. Hingegen wird Schritt (b') durchgeführt, wenn das erste Rezeptormolekül an eine Festphase gebunden ist.

Der Begriff "Analyt" bezeichnet im Sinne dieser Erfindung jedes Molekül, das in einer Probe, vorzugsweise einer biologischen Probe, vorkommt und durch ein Rezeptormolekül gebunden werden kann.

Der Begriff "Analytderivat" bezeichnet eine Substanz, mit der das gegen den Analyten gerichtete Rezeptormolekül kreuzreagiert.

Der Begriff "Rezeptormolekül" betrifft jedes Molekül, das eine spezifische Bindungsstelle für ein anderes Molekül, z.B. einen Analyten, aufweist. Beispiele für Rezeptormoleküle sind Hormonrezeptoren oder Antikörper. Die Rezeptormoleküle können natürlichen, rekombinanten, synthetischen oder semisynthetischen Ursprungs sein.

Das für das Analytderivat spezifische zweite Rezeptormolekül kann, muß jedoch nicht mit dem Analyten kreuzreagieren. Es ist üblicherweise vom ersten Rezeptormolekül verschieden. Darüber hinaus kann es eine Markierung tragen, die jedoch von der Markierung des ersten Rezeptormoleküls verschieden ist, sofern das erste Rezeptormolekül markiert ist.

Mit dem erfindungsgemäßen Verfahren lassen sich Analyten in einer Probe leicht qualitativ, wie auch quantitativ nachweisen. Dieses Ergebnis wird dadurch erreicht, daß das Analytderivat durch das zweite, dafür spezifische Rezeptormolekül maskiert wird und die Affinität des ersten Rezeptormoleküls für das Analytderivat somit herabgesetzt wird. Die Folge davon ist, daß das Reaktionsgleichgewicht im Inkubationsansatz in Richtung Analyt-analytspezifisches erstes Rezeptormolekül verschoben wird. Durch den Einsatz unterschiedlicher Analytderivat-spezifischer Rezeptormoleküle kann die Reaktivität des Analytderivats bzw. des Komplexes aus Analytderivat und Analytderivat-spezifischem zweiten Rezeptormolekül gegenüber dem Analyt-spezifischen ersten Rezeptormolekül auf einfache Weise variiert werden. Dadurch steht ein weiterer Freiheitsgrad zur Optimierung des Tests zur Verfügung.

Dem Fachmann ist bekannt, welche Materialien er als Festphasen verwenden kann und welche Bedingungen er für die Durchführung des erfindungsgemäßen Verfahrens einsetzt. Derartige Materialien und Bedingungen sind vorzugsweise üblicher Natur, wie z.B. in Harlow und Lane, "Antibodies, A Laboratory Manual", CSH-Press, Cold Spring Harbor, 1988 beschrieben. Darüber hinaus weiß der Fachmann, daß zwischen den einzelnen vorstehend genannten Schritten Waschschritte mit geeigneten Puffern durchzuführen sind. Auch hier dient beispielsweise das Dokument Harlow und Lane, a.a.O. als Leitfaden. Ferner ist dem Fachmann bekannt, wie er Zeit und Temperatur für die einzelnen, im erfindungsgemäßen Verfahren genannten Schritte wählt. Vorzugsweise liegt die Temperatur zwischen Raumtemperatur (üblicherweise etwa 22 °C) und 37 °C. Eine besonders bevorzugte Temperatur für die Inkubation ist 37 °C. Auch der Nachweis des an das Analytderivat gebundenen ersten Rezeptormoleküls oder des an das erste Rezeptormolekül gebundenen Analytderivats erfolgt nach üblichen Verfahren, beispielsweise durch Messung eines Signals, das von dem markierten Analytderivat oder dem markierten ersten Rezeptormolekül ausgesandt wird. Sofern weder Analytderivat noch erstes Rezeptormolekül markiert sind, kann der Nachweis mit einem zweiten, beispielsweise für das erste Rezeptormolekül spezifisch markierten Antikörper oder Antikörperfragment erfolgen.

In einer bevorzugten Ausführungsform des erfindungsgemäßen Verfahrens inkubiert man das Analytderivat vor der Inkubation mit der Probe und dem ersten Rezeptormolekül mit dem zweiten Rezeptormolekül.

Dieser erste Inkubationsschritt hat zur Folge, daß das Analytderivat bereits maskiert in den Inkubationsansatz eingeführt wird. Ein weiterer Vorteil liegt in der Tatsache, daß eine geringere Menge an Analyt-spezifischem zweiten Rezeptormolekül notwendig ist, um das Analytderivat vollständig oder nahezu vollständig zu maskieren. Sofern man beide Rezeptormoleküle gleichzeitig mit dem Analytderivat inkubiert, konkurrieren diese um die verfügbaren Bindungsstellen am Analytderivat. Dies hat zur Folge, daß zur Durchführung des Tests eine größere Menge an zweitem Rezeptormolekül eingesetzt werden muß.

In einer weiteren bevorzugten Ausführungsform des erfindungsgemäßen Verfahrens ist das erste Rezeptormolekül markiert. In dieser Ausführungsform dient die Markierung des ersten Rezeptormoleküls zum Nachweis des Analyten.

In einer weiteren bevorzugten Ausführungsform des erfindungsgemäßen Verfahrens ist das Analytderivat markiert.

Die Markierung des Rezeptormoleküls bzw. des Analytderivaten kann vielfältiger Natur sein. Besonders bevorzugt sind erfindungsgemäß eine radioaktive, chemilumineszierende, biolumineszierende, fluoreszierende, phosphoreszierende, elektrolumineszierende Markierung, ein Enzym, Biotin oder eine zur Absorption befähigte Gruppe.

Eine weitere bevorzugte Ausführungsform des erfindungsgemäßen Verfahrens ist dadurch gekennzeichnet, daß das Analytderivat an eine Festphase gebunden wird. In dieser Ausführungsform ist das Analytderivat vorzugsweise nicht markiert. Beispiele für im erfindungsgemäßen Verfahren verwendbare Festphasen sind Mikrotiterplatten, vorzugsweise aus Polystyrol, Polymerkügelchen, Röhrchen oder Membranen.

In einer weiteren bevorzugten Ausführungsform des erfindungsgemäßen Verfahrens inkubiert man das Analytderivat nach der Bindung an die Festphase mit dem zweiten Rezeptormolekül. Diese Ausführungsform hat den besonderen Vorteil, daß zunächst mit dem Analytderivaten beschichtete Festphasen, z.B. Mikrotiterplatten, hergestellt werden können, die auch vor einem Einsatz im erfindungsgemäßen analytischen Verfahren längerfristig unter üblichen Bedingungen gelagert werden können.

Eine weitere bevorzugte Ausführungsform des erfindungsgemäßen Verfahrens ist dadurch gekennzeichnet, daß das erste Rezeptormolekül an eine Festphase gebunden wird.

In einer weiteren bevorzugten Ausführungsform des erfindungsgemäßen Verfahrens wird das Analytderivat nach der Bindung des ersten Rezeptormoleküls an die Festphase mit dem zweiten Rezeptormolekül inkubiert.
Auch diese Ausführungsform hat den besonderen Vorteil, daß die Festphasen nach Bindung des ersten Rezeptormoleküls vor einem Einsatz im erfindungsgemäßen analytischen Verfahren längerfristig unter geeigneten Bedingungen aufbewahrt werden können.

Bevorzugt ist ferner eine Ausführungsform, in der das erste und das zweite Rezeptormolekül mit Ausnahme der Markierung identisch sind.

Ferner bevorzugt ist eine Ausführungsform in der das erste und das zweite Rezeptormolekül verschieden sind. Üblicherweise wird man im erfindungsgemäßen Test zwei verschiedene Antikörper verwenden.

In einer weiteren bevorzugten Ausführungsform ist das erste und/oder das zweite Rezeptormolekül ein Antikörper oder ein Antikörperfragment.
Der hier verwendete Antikörper kann ein polyclonaler, ein monoclonaler, ein chimärer oder ein synthetisch hergestellter Antikörper sein. Die Herstellung derartiger Antikörper ist im Stand der Technik bekannt und z.B. in Harlow und Lane, a.a.O. beschrieben. Antikörperfragmente sind ebenfalls im Stand der Technik bekannt. Im Sinne der Erfindung ist allenfalls notwendig, daß sie das Analytderivat bzw. das Analyt spezifisch binden können und, sofern sie als erstes Rezeptormolekül verwendet werden, mit einer geeigneten Markierung versehen werden können. Beispiele für Antikörperfragmente sind Fv, Fab oder F(ab)₂-Fragmente.
In einer weiteren bevorzugten Ausführungsform des erfindungsgemäßen Verfahrens ist der Analyt ein Ligand. Unter "Ligand" wird erfindungsgemäß jedes Molekül verstanden, für das ein dieses bindender natürlicherweise vorkommender Rezeptor existiert. Vorzugsweise ist der Ligand ein natürlicherweise auftretendes Molekül und besonders bevorzugt tritt dieses Molekül in Körperflüssigkeiten, beispielsweise in Serum auf.

In einer besonders bevorzugten Ausführungsform des erfindungsgemäßen Verfahrens ist der Ligand Thyroxin, Triiodthyronin oder ein Steroid, beispielsweise Estradiol.

In einer weiteren bevorzugten Ausführungsform des erfindungsgemäßen Verfahrens wird vor Schritt (a) eine Präinkubation des Analyten mit dem ersten Rezeptormolekül durchgeführt.
Mit dieser Ausführungsform wird erreicht, daß eine wesentliche Menge an freiem Analyten, insbesondere Liganden gebunden wird, was wiederum günstige Auswirkungen auf den quantitativen Nachweis dieses Liganden im erfindungsgemäßen Testsystem nach sich zieht.

Vorzugsweise wird der Präinkubationsschritt 5 bis 30 Minuten durchgeführt.

Die Erfindung betrifft ferner einen Kit, der mindestens enthält,
(a) ein Analytderivat; und/oder
(b) ein markiertes Analytderivat; und
(c) ein erstes, für einen Analyten und das Analytderivat spezifisches Rezeptormolekül; und/oder
(d) ein erstes, für einen Analyten und das Analytderivat spezifisches markiertes Rezeptormolekül; und
(e) ein zweites Rezeptormolekül, das spezifisch das Analytderivat oder das Analytderivat und den Analyten bindet.

Der Fachmann kann den erfindungsgemäßen Kit durch übliche weitere Komponenten, z.B. geeignete Puffer ergänzen.

Schließlich betrifft die Erfindung die Verwendung des erfindungsgemäßen Kits zur Durchführung des erfindungsgemäßen Verfahrens.
Die folgenden Beispiele erläutern die Erfindung.

### Beispiel 1

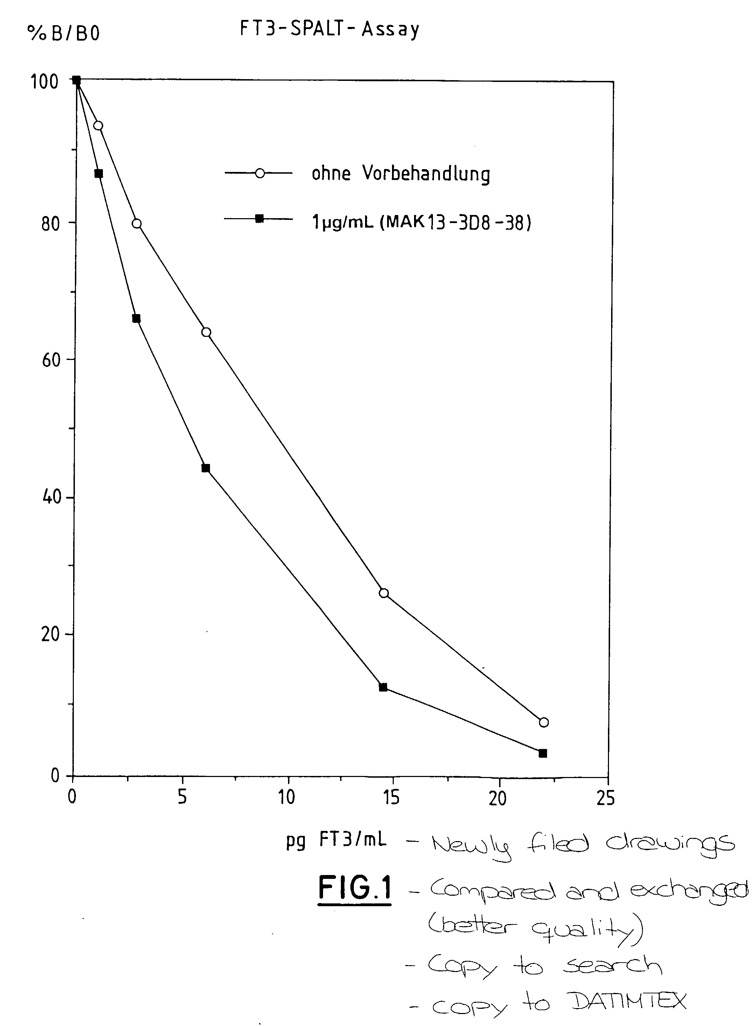

In BeriLux ® T3 (Behringwerke AG, Marburg) beschichteten Röhrchen (Röhrchen, die mit einem Protein-T3-Konjugat beschichtet sind) werden 0,5 ml einer Anti-T3-Antikörper-Lösung (1 µg unmarkierter BeriLux® T3-Tracerantikörper/ml Inkubationspuffer) gegeben. Nach einer 2stündigen Inkubation bei 37 °C wird die Lösung entfernt und die Röhrchen werden einmal mit 1 ml Waschpuffer gewaschen. Die so vorbehandelten Röhrchen werden als Festphase in einem FT3-Assay mit markiertem Anti-T3-Antikörper als Tracer eingesetzt. Die erzielte Signaldynamik ist höher als bei den unbehandelten Röhrchen (Fig. 1).

### Inkubationspuffer:

0,1 M Phosphat-Puffer, pH 7,4 mit 1 g Rinder-IgG, 8 g NaCl und 1 g Natriumazid pro Liter.

### Tracer:

30 ng BeriLux® T3-Tracerantikörper pro ml 0,1 M Phosphat-Puffer, pH 7,4 mit 1 g Rinder-IgG, 8 g NaCl und 1 g Natriumazid pro Liter (BeriLux ® T3-Tracerantikörper = MAK 13-3D8-38; Hersteller: Immunogen International Ltd., United Kingdom)

### Waschpuffer:

0,1 M Phosphat-Puffer, pH 7,4 mit 8 g NaCl pro Liter.

### Testbedingungen:

100 ml Probe (Standard bzw. Serum) und 200 µl Tracer werden 60 Minuten bei Raumtemperatur geschüttelt. Nach viermaligem Waschen mit je 1 ml Waschpuffer wird die Signalaktivität im BeriLux® Analysegerät gemessen.

### Beispiel 2

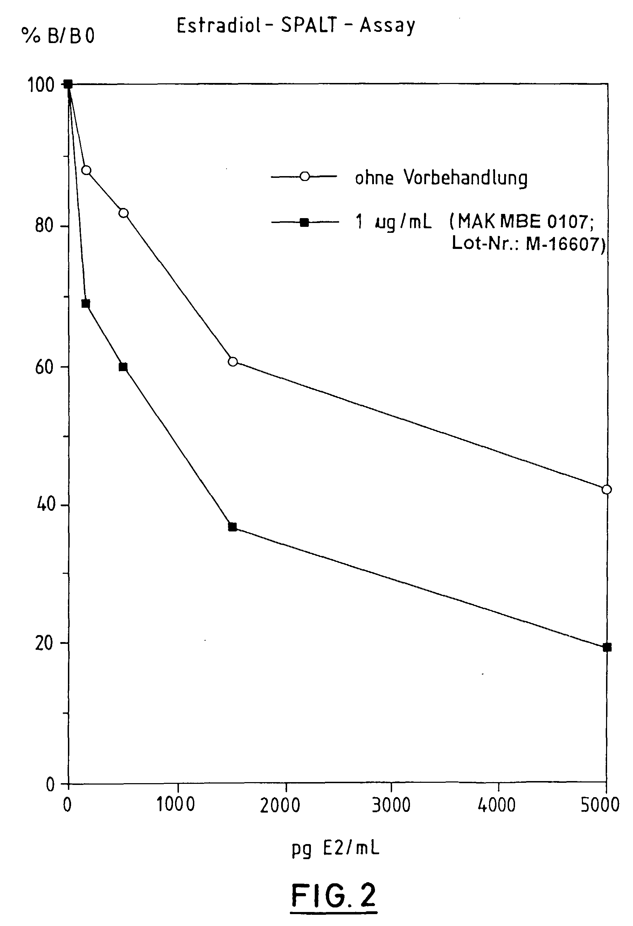

1 mg Magnetpartikel, die mit einem IgG-Estradiol-Konjugat beschichtet wurden, werden mit 8 ml einer Lösung in Kontakt gebracht, die 1 µg Anti-Estradiol-Anti-körper (Medix-Biotech, Cat.No. MBE 0107; Lot-Nr. M-16607)/ml Inkubationspuffer enthält. Nach einer einstündigen Inkubation bei 37 °C werden die Magnetpartikel gewaschen und als Festphase in einem Estradiol-SPALT-Test eingesetzt. Als Tracer dient ein mit BeriLux^{®}-Label markierter Anti-Estradiol-Antikörper (BiosPacific, Clone No. A 54010014 P). Die mit den derart behandelten Magnetpartikeln erhaltene Standardkurve besitzt eine höhere Signaldynamik als die, die mit der nicht nachbehandelten Festphase unter gleichen Bedingungen erzielt wird (Fig. 2).

### Inkubationspuffer:

0,1 M Tris/HCl-Puffer, pH 8,0 mit 8,7 g NaCl, 1 g Rinder-IgG, 1 g Tween^{®} 20 und 1 g Natriumazid pro Liter.

### Tracer:

10 ng Anti-Estradiol-Antikörper (BiosPacific) pro ml 0,1 M Phosphat-Puffer, pH 6,3 mit 5,9 g NaCI, 1 g Rinder-IgG, 1 g Natriumazid, 15 mg Danazol und 15 mg Hydrocortison pro Liter.

### Waschpuffer:

0,1 M Phosphat-Puffer, pH 7,4 mit 8,0 g NaCl pro Liter.

### Testbedingungen:

20 µl Magnetpartikelsuspension (25 µg beschichtete Magnetpartikel) + 100 µl Probe (Standard bzw. Serum) und 200 µl Tracer werden 30 Minuten bei 37°C inkubiert. Nach dreimaligem Waschen mit je 0,5 ml Waschpuffer wird die Signalaktivität im BeriLux^{®} Analysegerät gemessen

Die Figuren zeigen:
**Fig.1**
   FT3-Lumineszenzimmunoassay nach dem SPALT-Verfahren. Als Festphase dienen mit Protein -T3-Konjugat (=Analytderivat) beschichtete Röhrchen (obere Kurve). Durch Maskierung des Analytderivates mit einem Anti T3 Antikörper erhält man eine Standardkurve mit höherer Signaldynamik (untere Kurve)
**Fig. 2**
   SPALT-Assay zur Bestimmung von Estradiol. Als Festphase dienen mit Protein-Estradiol-Konjugat (=Analytderivat) beschichtete Röhrchen (obere Kurve). Durch Maskierung des Analytderivats mit einem Anti-Estradiol-Antikörper erhält man eine Standardkurve mit höherer Signaldynamik (untere Kurve).

## Patentansprüche

1. Verfahren zum Nachweis eines Analyten in einem kompetitiven Immuntest, dadurch gekennzeichnet, daß man folgende Schritte durchführt:
a) Inkubation eines Analytderivats mit einer möglicherweise den Analyten enthaltenden Probe sowie einem für Analyt und Analytderivat spezifischen ersten Rezeptormolekül, wobei der Inkubationsansatz ferner ein zweites Rezeptormolekül enthält, das spezifisch das Analytderivat oder das Analytderivat und den Analyten bindet und wobei entweder das Analytderivat oder das erste Rezeptormolekül festphasengebunden vorliegt;
b) Abtrennung des nicht an das Analytderivat gebundenen ersten Rezeptormoleküls, sofern das Analytderivat festphasengebunden vorliegt; oder
b') Abtrennung des nicht an das erste Rezeptormolekül gebundenen Analytderivats, sofern das erste Rezeptormolekül festphasengebunden vorliegt; und
c) Detektion des an das Analytderivat gebundenen ersten Rezeptormoleküls oder des an das erste Rezeptormolekül gebundenen Analytderivats.

2. Verfahren nach Anspruch 1, dadurch gekennzeichnet, daß man das Analytderivat mit dem zweiten Rezeptormolekül vor der Inkubation mit der Probe und dem ersten Rezeptormolekül inkubiert.

3. Verfahren nach Anspruch 1 oder 2, dadurch gekennzeichnet, daß das erste Rezeptormolekül markiert ist.

4. Verfahren nach Anspruch 1 oder 2, dadurch gekennzeichnet, daß das Analytderivat markiert ist.

5. Verfahren nach Anspruch 3 oder 4, dadurch gekennzeichnet, daß die Markierung eine radioaktive, chemilumineszierende, biolumineszierende, fluoreszierende, phosphoreszierende, elektrolumineszierende Markierung, ein Enzym, Biotin, oder eine zur Absorption befähigte Gruppe ist.

6. Verfahren nach einem der Ansprüche 1 bis 3 und 5, dadurch gekennzeichnet, daß das Analytderivat an eine Festphase gebunden wird.

7. Verfahren nach einem der Ansprüche 1 bis 3, 5 und 6, dadurch gekennzeichnet, daß man das Analytderivat nach der Bindung an die Festphase mit dem zweiten Rezeptormolekül inkubiert.

8. Verfahren nach einem der Ansprüche 1, 2, 4 und 5, dadurch gekennzeichnet, daß das erste Rezeptormolekül an eine Festphase gebunden wird.

9. Verfahren nach einem der Ansprüche 1, 2, 4, 5 und 8, dadurch gekennzeichnet, daß das Analytderivat nach der Bindung des ersten Rezeptormoleküls an die Festphase mit dem zweiten Rezeptormolekül inkubiert wird.

10. Verfahren nach einem der Ansprüche 1 bis 9, dadurch gekennzeichnet, daß das erste und das zweite Rezeptormolekül mit Ausnahme der Markierung identisch sind.

11. Verfahren nach einem der Ansprüche 1 bis 9, dadurch gekennzeichnet, daß das erste und das zweite Rezeptormolekül verschieden sind.

12. Verfahren nach einem der Ansprüche 1 bis 11, dadurch gekennzeichnet, daß das erste und/oder das zweite Rezeptormolekül ein Antikörper oder ein Antikörperfragment ist/sind.

13. Verfahren nach einem der Ansprüche 1 bis 12, dadurch gekennzeichnet, daß der Analyt ein Ligand ist.

14. Verfahren nach Anspruch 13, dadurch gekennzeichnet, daß der Ligand Thyroxin, Triiodthyronin oder ein Steroid ist.

15. Verfahren nach einem der Ansprüche 1 bis 14, dadurch gekennzeichnet, daß man vor Schritt (a) eine Präinkubation des Analyten mit dem ersten Rezeptormolekül durchführt.

16. Kit zum Nachweis eines Analyten mittels eines kompetitiven Immuntests, mindestens enthaltend:
a) ein Analytderivat; und/oder
b) ein markiertes Analytderivat;
c) ein erstes, für einen Analyten und das Analytderivat spezifisches Rezeptormolekül; und/oder
d) ein erstes, für einen Analyten und das Analytderivat spezifisches markiertes Rezeptormolekül;
e) ein zweites Rezeptormolekül, das spezifisch das Analytderivat oder das Analytderivat und den Analyten bindet.

17. Verwendung eines Kits nach Anspruch 16 zur Durchführung des Verfahrens nach einem der Ansprüche 1 bis 15.

## Claims

1. A method for the detection of an analyte in a competitive immunoassay, which comprises carrying out the following steps:
(a) incubation of an analyte derivative with a sample possibly containing the analyte and a first receptor molecule specific for analyte and analyte derivative, the incubation mixture also containing a second receptor molecule which specifically binds the analyte derivative or the analyte derivative and the analyte and either the analyte derivative or the first receptor molecule being bound to the solid phase;
(b) separation of the first receptor molecule not bound to the analyte derivative if the analyte derivative is bound to the solid phase; or
(bN) separation of the analyte derivative not bound to the first receptor molecule if the first receptor molecule is bound to the solid phase; and
(c) detection of the first receptor molecule bound to the analyte derivative or of the analyte derivative bound to the first receptor molecule.

2. The method as claimed in claim 1, wherein the analyte derivative is incubated with the second receptor molecule before incubation with the sample and the first receptor molecule.

3. The method as claimed in claim 1 or 2, wherein the first receptor molecule is labeled.

4. The method as claimed in claim 1 or 2, wherein the analyte derivative is labeled.

5. The process as claimed in claim 3 or 4, wherein the label is a radioactive, chemiluminescent, bioluminescent, fluorescent, phosphorescent or electroluminescent label, an enzyme, biotin, or a group capable of absorption.

6. The method as claimed in one of claims 1 to 3 and 5, wherein the analyte derivative is bound to a solid phase.

7. The method as claimed in one of claims 1 to 3, 5 and 6, wherein the analyte derivative is incubated with the second receptor molecule after binding to the solid phase.

8. The method as claimed in one of claims 1, 2, 4 and 5, wherein the first receptor molecule is bound to a solid phase.

9. The method as claimed in one of claims 1, 2, 4, 5 and 8, wherein the analyte derivative is incubated with the second receptor molecule after binding the first receptor molecule to the solid phase.

10. The method as claimed in one of claims 1 to 9, wherein the first and the second receptor molecule are identical with the exception of the label.

11. The method as claimed in one of claims 1 to 9, wherein the first and the second receptor molecule are different.

12. The method as claimed in one of claims 1 to 11, wherein the first and/or the second receptor molecule is/are an antibody or an antibody fragment.

13. The method as claimed in one of claims 1 to 12, wherein the analyte is a ligand.

14. The method as claimed in claim 13, wherein the ligand is thyroxine, triiodothyronine or a steroid.

15. The method as claimed in one of claims 1 to 14, wherein a preincubation of the analyte with the first receptor molecule is carried out before step (a).

16. A kit for the detection of an analyte by means of a competitive immunoassay containing at least
(a) an analyte derivative; and/or
(b) a labeled analyte derivative;
(c) a first receptor molecule specific for an analyte and the analyte derivative; and/or
(d) a first labeled receptor molecule specific for an analyte and the analyte derivative;
(e) a second receptor molecule which specifically binds the analyte derivative or the analyte derivative and the analyte.

17. The use of a kit as claimed in claim 16 for carrying out the method as claimed in one of claims 1 to 15.

## Revendications

1. Procédé pour la détection d'un analyte dans un essai immunologique compétitif, caractérisé en ce que l'on effectue les étapes suivantes :
(a) incubation d'un dérivé d'analyte avec un échantillon contenant éventuellement l'analyte, ainsi qu'une première molécule réceptrice spécifique de l'analyte et du dérivé d'analyte, le mélange d'incubation contenant en outre une seconde molécule réceptrice qui se lie spécifiquement au dérivé d'analyte ou au dérivé d'analyte et à l'analyte, et soit le dérivé d'analyte, soit la première molécule réceptrice étant fixé à une phase solide ;
(b) séparation de la première molécule réceptrice non liée au dérivé d'analyte, lorsque le dérivé d'analyte est fixé à une phase solide ; ou
(b') séparation du dérivé d'analyte non lié à la première molécule réceptrice, lorsque la première molécule réceptrice est fixée à une phase solide ; et
(c) détection de la première molécule réceptrice liée au dérivé d'analyte ou du dérivé d'analyte lié à la première molécule réceptrice.

2. Procédé selon la revendication 1, caractérisé en ce l'on met le dérivé d'analyte à incuber avec la seconde molécule réceptrice, avant l'incubation avec l'échantillon et la première molécule réceptrice.

3. Procédé selon la revendication 1 ou 2, caractérisé en ce que la première molécule réceptrice est marquée.

4. , Procédé selon la revendication 1 ou 2, caractérisé en ce que le dérivé d'analyte est marqué.

5. Procédé selon la revendication 3 ou 4, caractérisé en ce que le marqueur est un marqueur radioactif, chimiluminescent, bioluminescent, fluorescent, phosphorescent, électroluminescent, une enzyme, la biotine ou un groupe apte à l'absorption.

6. Procédé selon l'une des revendications 1 à 3 et 5, caractérisé en ce que le dérivé d'analyte est fixé à une phase solide.

7. Procédé selon l'une des revendications 1 à 3, 5 et 6, caractérisé en ce que l'on met le dérivé d'analyte à incuber avec la seconde molécule réceptrice après la dation à la phase solide.

8. Procédé selon l'une des revendications 1, 2, 4 et 5, caractérisé en ce que la première molécule réceptrice est fixée à une phase solide.

9. Procédé selon l'une des revendications 1, 2, 4, 5 et 8, caractérisé en ce que le dérivé d'analyte est mis à incuber avec la seconde molécule réceptrice après la fixation de la première molécule réceptrice à la phase solide.

10. Procédé selon l'une des revendications 1 à 9, caractérisé en ce que la première molécule réceptrice et la seconde sont identiques, à l'exception du marquage.

11. Procédé selon l'une des revendications 1 à 9, caractérisé en ce que la première molécule réceptrice et la seconde sont différentes.

12. Procédé selon l'une des revendications 1 à 11, caractérisé en ce que la première molécule réceptrice et/ou la seconde est/sont un anticorps ou un fragment d'anticorps.

13. Procédé selon l'une des revendications 1 à 12, caractérisé en ce que l'analyte est un ligand.

14. Procédé selon la revendication 13, caractérisé en ce que le ligand est la thyroxine, la triodothyronine ou un stéroïde.

15. Procédé selon l'une des revendications 1 à 14, caractérisé en ce que l'on effectue avant l'étape (a) une préincubation de l'analyte avec la première molécule réceptrice.

16. Nécessaire pour la détection d'un analyte à l'aide d'un essai immunologique compétitif, contenant au moins :
(a) un dérivé d'analyte ; et/ou
(b) un dérivé d'analyte marqué ;
(c) une première molécule réceptrice spécifique d'un analyte et du dérivé d'analyte ; et/ou
(d) une première molécule réceptrice marquée spécifique d'un analyte et du dérivé d'analyte ; et
(e) une seconde molécule réceptrice qui se lie spécifiquement au dérivé d'analyte ou au dérivé d'analyte et à l'analyte.

17. Utilisation d'un nécessaire selon la revendication 16, pour la mise en oeuvre du procédé selon l'une des revendications 1 à 15.
